# FASCICULE DE BREVET EUROPEEN

(11) **EP 2 024 774 B1**
(45) Date de publication et mention de la délivrance du brevet: **10.05.2017**
(21) Numéro de dépôt: 07731440.9
(22) Date de dépôt: 11.05.2007
(51) Int. Cl.: G02B 23/26, G02B 6/06, A61B 1/00

(54) **DISPOSITIF ET PROCEDE D'ENDOSCOPIE POUR UNE OBSERVATION SIMULTANEE DE PLUSIEURS ZONES D'INTERET**
ENDOSKOPIEEINRICHTUNG UND VERFAHREN ZUR GLEICHZEITIGEN BEOBACHTUNG MEHRERER INTERESSIERENDER ZONEN
ENDOSCOPY DEVICE AND METHOD FOR SIMULTANEOUS OBSERVATION OF SEVERAL ZONES OF INTEREST

(30) Priorité: 12.05.2006 FR 0604259
(43) Date de publication de la demande: 18.02.2009
(73) Titulaire: Mauna Kea Technologies, 75010 Paris (FR)
(72) Inventeur: LACOMBE, François, 92370 Chaville (FR); VIELLEROBE, Bertrand, 94130 Nogent sur Marne (FR); BOULAROT, Nicolas, 94500 Champigny sur Marne (FR); DOUSSOUX, François, 75009 Paris (FR); PERCHANT, Aymeric, 94120 Fontenay sous Bois (FR)
(74) Mandataire: Pontet Allano & Associes
(86) Numéro de dépôt international: PCT/FR2007/000799
(87) Numéro de publication internationale: WO 2007/132085

(56) Documents cités:
- EP-A- 1 586 855
- US-A- 5 823 942
- US-A1- 2004 037 554
- US-B1- 6 826 422
- GMITRO A F ET AL: "CONFOCAL MICROSCOPY THROUGH A FIBER-OPTIC IMAGING BUNDLE", OPTICS LETTERS, OSA, OPTICAL SOCIETY OF AMERICA, WASHINGTON, DC, US, vol. 18, no. 8, 15 April 1993 (1993-04-15) , pages 565-567, XP000364196, ISSN: 0146-9592
- FLUSBERG BENJAMIN A ET AL: "Fiber-optic fluorescence imaging", NATURE METHODS, NATURE PUBLISHING GROUP, GB, vol. 2, no. 12, 1 December 2005 (2005-12-01), pages 941-950, XP008084282, ISSN: 1548-7091, DOI: 10.1038/NMETH820
- E. LAEMMEL: '"Fibered Confocal Fluorescence Microscopy (Cell-viZio TM) Facilitates Extended Imaging in the Filed of Microcirculation"' J. OF VASCULAR RESEARCH 2004 30 Septembre 2004, ONLINE,
- C. P. LIN ET AL.: '"Fiber-coupled multiplexed confocal microscope"' OPTICS LETTERS vol. 25, no. 13, 01 Juillet 2000,

## Description

### Domaine technique

La présente invention concerne un dispositif d'endoscopie.

Un dispositif selon l'invention peut être appliqué de manière non limitative à des domaines tels que la spectroscopie, l'imagerie in vivo et in situ, l'imagerie de fluorescence, l'imagerie de réflectance, l'imagerie multiphotonique, et la micro-endoscopie.

### Etat de la technique antérieure

De manière générale, un dispositif d'endoscopie comprend un guide de lumière comprenant une unique fibre optique ou un toron d'une pluralité de fibres optiques. Une première extrémité du guide, que l'on appellera par la suite « extrémité distale », est prévue pour être insérée dans un corps étudié ou plaquée contre le corps, de manière à pouvoir examiner ledit corps. On appellera par la suite « extrémité proximale » l'autre extrémité du guide. Le guide permet une certaine souplesse d'insertion, et permet de transporter un faisceau lumineux depuis l'extrémité proximale jusqu'à l'extrémité distale du guide ou inversement.

Le dispositif d'endoscopie peut comprendre en outre une tête optique connectée à l'extrémité distale du guide. La tête optique permet de focaliser en surface ou en profondeur du corps examiné un faisceau lumineux d'excitation provenant du guide. La tête optique permet en outre de collecter un faisceau lumineux et de transmettre ce faisceau au guide. Le guide et la tête optique constituent alors un microscope confocal miniature. Pour pouvoir observer tout un champ d'observation, le faisceau d'excitation focalisé peut être balayé dans le champ par des moyens de balayage situés du côté distal ou proximal du guide.

Quand les moyens de balayage sont situés coté proximal, le guide de lumière a pour fonction de transporter, point à point, une image. Il doit alors être constitué d'une pluralité de fibres optique. On parle alors de guide d'image.

Un examen microscopique in situ et in vivo réalisé avec un tel dispositif ne permet une observation d'une zone d'intérêt que sur un champ d'observation très réduit, typiquement quelques centaines de micromètres de côté environ. Cette limitation peut constituer un problème pour l'interprétation de phénomènes se manifestant sur une région plus grande que le champ observé, ou ne pouvant être interprétés que si le champ observé est plus grand que la région dans laquelle se produit le phénomène. Cette limitation est aussi un problème quand le phénomène étudié se produit dans des zones d'intérêt plus ou moins éloignées les une des autres, notamment à des distances grandes devant tout champ raisonnablement observable.

Offrir la possibilité d'observer plusieurs zones d'intérêt avec une résolution suffisante permettrait de résoudre cette limitation. Par exemple, dans le cas de l'imagerie du système nerveux chez le petit animal, différentes régions du cerveau ou bien différentes terminaisons nerveuses, ou les deux, pourraient être observées à des échelles microscopiques. Un autre exemple consiste en l'étude des phénomènes vasculaires, où un réseau sanguin pourrait être l'objet d'une observation multiple de plusieurs sites.

Les documents US 5 823 942 A et US 6 826 422 B1 décrivent chacun un dispositif comprenant plusieurs guides de lumière comprenant chacun deux extrémités respectivement proximale et distale reliées par une ou plusieurs fibres optiques, les extrémités distales des guides étant confondues et étant donc placées au niveau d'une unique zone d'intérêt d'un échantillon.

Le document US 2004 037 554 A1 décrit un dispositif comprenant un unique toron de fibres, ledit toron comprenant plusieurs fibres optiques dont les extrémités distales sont toutes reliées et dont les extrémités proximales sont toutes reliées.

Le document EP 1 586 855 A décrit un dispositif comprenant :
- plusieurs guides de lumière comprenant chacun deux extrémités respectivement proximale et distale reliées par une ou plusieurs fibres optiques, les extrémités distales des guides étant séparées, et
- un interrupteur à cristal liquide à travers lequel on éclaire, de façon sélective, plusieurs fibres optiques.

Le document "confocal microscopy through a fiber-optic imaging bundle" de Arthur F. Gmitro et al. (Optics letters vol. 18 (1993)) décrit un microscope confocal comprenant un guide d'image et un système de balayage de l'extrémité proximale de ce guide.

Le document "Fiber-optic fluorescence imaging" de Benjamin A. Flusberg et al. (Nature methods, vol. 2 n°12) décrit différenttes Techniques d'imagerie par fibre optique, dont un mode confocal à guide d'image et balayage proximal.

Le but de la présente invention est de proposer un dispositif d'endoscopie offrant la possibilité d'observer simultanément ou quasi simultanément plusieurs zones d'intérêt.

### Exposé de l'invention

Cet objectif est atteint avec un dispositif selon la revendication 1.

Ainsi, le même système de balayage peut diriger un faisceau lumineux d'excitation dans plus d'un guide de lumière, de sorte que plusieurs guides de lumière profitent du même système de balayage. L'éclairement des extrémités proximales des fibres une par une est transmis par celles-ci jusqu'à leurs extrémités distales, où chaque fibre devient tour à tour une source d'illumination. Chaque guide de lumière est une sonde dont l'extrémité distale indépendante peut être implantée simultanément à d'autres sondes dans ou sur une zone d'intérêt différente d'un objet, système ou animal étudié. Le dispositif selon l'invention permet donc par exemple de réaliser de l'imagerie in vivo, in situ et simultanée de plusieurs zones d'intérêt distantes d'un même animal. Les dimensions des guides de lumière peuvent être suffisamment petites (typiquement quelques centaines de micromètres de diamètre) pour que chaque guide de lumière constitue une sonde micro-endoscopique peu encombrante. Le nombre de fibres et la dimension des fibres peuvent varier d'un guide à l'autre.

Le système de balayage peut consister en n'importe quel système classiquement utilisé pour balayer une surface d'entrée d'un guide d'image. Le système de balayage peut par exemple comprendre deux miroirs distincts, assurant le balayage horizontal et vertical de la surface d'entrée des guides de lumière.

Dans un mode de réalisation de dispositif selon l'invention, les guides de lumière peuvent être agencés pour conserver à l'extrémité proximale du toron de guides un ordonnancement des fibres d'un guide à l'autre, notamment lorsque le système de balayage ne fonctionne qu'avec des fibres ordonnées à l'extrémité proximale du toron de guides. Dans un autre mode de réalisation de dispositif selon l'invention, l'ordonnancement des fibres d'un guide à l'autre à l'extrémité proximale du toron de guides peut être quelconque, notamment lorsque le système de balayage s'accommode de fibres aléatoirement distribuées à l'extrémité proximale du toron de guides.

Le dispositif selon l'invention peut comprendre en outre une source de lumière pour émettre le faisceau lumineux d'excitation.

Le dispositif selon l'invention peut comprendre en outre une tête optique disposée sur une extrémité distale d'un des guides de lumière. La tête optique peut notamment permettre de focaliser dans un plan focal le faisceau lumineux d'excitation transmis à la tête optique par une fibre dudit un des guides, et peut permettre en outre de collecter un faisceau lumineux réfléchi ou émis sensiblement dans ce plan focal et de transmettre le faisceau collecté à la même fibre. Selon la tête optique utilisée, le faisceau d'excitation peut être focalisé en surface ou en profondeur. Le dispositif selon l'invention peut comprendre en outre des moyens pour construire une image à partir de l'ensemble de faisceaux collectés.

Les moyens d'extraction peuvent permettre de regrouper des faisceaux lumineux collectés en fonction du guide de lumière collectant les faisceaux. Pour chaque guide de lumière, l'image construite correspond alors à un champ d'observation d'une zone d'intérêt. La taille du champ d'observation et la profondeur d'observation dépendent du nombre et du diamètre des fibres dans le guide, et éventuellement des caractéristiques de la tête optique disposée sur l'extrémité distale du guide. Le dispositif selon l'invention peut comprendre en outre des moyens pour visualiser l'image.

De manière générale, un dispositif selon l'invention peut comprendre en outre des moyens pour acquérir et traiter une image et qui mettent en oeuvre un procédé tel que décrit dans la demande de brevet WO 2004/010377 A1.

Le dispositif selon l'invention peut comprendre en outre des moyens pour varier le contenu spectral du faisceau lumineux d'excitation en fonction du guide de lumière dans lequel est dirigé le faisceau lumineux d'excitation. De manière préférentielle, on peut associer ainsi une longueur d'onde différente à chaque guide de lumière. Les moyens de variation du contenu spectral et le système de balayage associés constituent des moyens de balayage multicouleur, qui peuvent permettre d'exciter simultanément des colorants différents présents dans des zones d'intérêt différentes.

Grâce aux Tables de correspondance, aucune conservation d'ordonnancement des fibres n'est requise d'un guide à l'autre pour construire des images, ce qui représente un réel avantage dans la mesure où cela relâche beaucoup les contraintes de couplage des guides au système de balayage, et les contraintes d'ordonnancement des fibres d'un guide à l'autre par exemple lorsqu'ils sont montés en un toron de guides.

Dans le dispositif selon l'invention, le faisceau lumineux d'excitation peut être monochromatique ou polychromatique, et peut être continu ou pulsé. Le dispositif selon l'invention peut être appliqué à de l'imagerie de fluorescence, de réflectance, ou encore multiphotonique.

### Description des figures et modes de réalisation

D'autres avantages et caractéristiques de l'invention apparaîtront à l'examen de la description détaillée de mises en oeuvre nullement limitatives, et des dessins annexés suivants :
- la figure 1 illustre un premier mode de réalisation de dispositif selon l'invention,
- la figure 2 illustre une vue en coupe d'un des guides d'image du premier mode de réalisation de dispositif selon l'invention,
- la figure 3 illustre une vue en coupe d'un toron de guides d'image du premier mode de réalisation de dispositif selon l'invention,
- la figure 4 illustre une vue en coupe d'un toron de guides d'image d'un second mode de réalisation de dispositif selon l'invention,
- la figure 5 illustre un balayage d'un premier mode de réalisation de procédé,
- la figure 6 illustre un balayage d'un second mode de réalisation de procédé, et
- la figure 7 illustre un balayage d'un troisième mode de réalisation de procédé

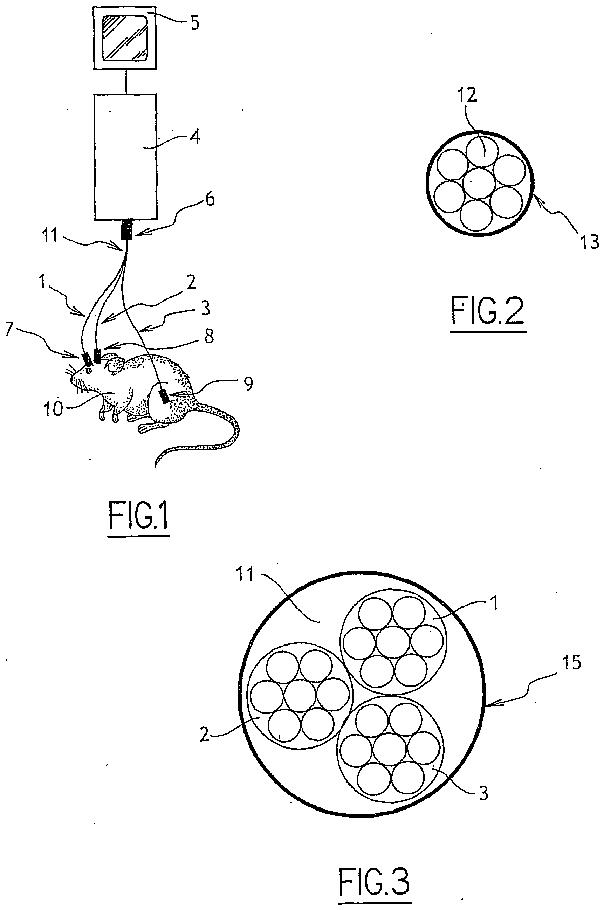

On va tout d'abord décrire, en référence aux figures 1, 2 et 3, un premier mode de réalisation de dispositif selon l'invention, qui est un mode de réalisation préférentiel de dispositif selon l'invention.

Le premier mode de réalisation de dispositif selon l'invention illustré sur la figure 1 comprend trois guides d'image 1, 2, 3, un boîtier 4 comprenant un système de balayage, et un écran de visualisation 5.

La figure 2 illustre une vue en coupe de l'extrémité distale d'un des guides d'image. Chaque guide d'image est sensiblement identique et consiste en un toron de fibres optiques, comprenant plusieurs milliers de coeurs de fibres optiques 12 non ordonnées de manière régulière. Il n'y a donc pas de conservation de l'ordonnancement des fibres d'un guide 1, 2 ou 3 à l'autre à l'extrémité proximale du toron 11 de guides. A leur extrémité distale, chaque guide d'image est en outre entouré par une gaine de protection 13. Le diamètre des fibres est de quelques micromètres, et le diamètre des guides est de quelques centaines de micromètres. Pour plus de clarté, on a représenté un nombre limité de fibres sur la figure 2, et on représentera de même un nombre limité de fibres par guide sur les figures suivantes. L'extrémité distale de chaque guide d'image est indépendante et séparée des extrémités distales des autres guides d'image. Chaque extrémité distale de guide est donc mobile indépendamment des autres guides d'image, et les extrémités distales des guides d'image peuvent donc être implantées simultanément dans différentes zones d'intérêt d'un animal 10.

Les extrémités proximales des guides d'image sont associées en un toron 11 de guides d'image. La figure 3 illustre une vue en coupe du toron 11 de guides d'image. Pour fabriquer le toron 11 de guides, les extrémités proximales des trois guides d'image 1, 2, 3 ont été séparées de leur gaine de protection 13 puis collées ensemble. Le tout est entouré par une gaine de protection 15. L'extrémité proximale du toron 11 de guides regroupe les extrémités proximales des guides, qui forment ainsi une surface d'entrée de balayage pour un faisceau lumineux d'excitation. Le toron 11 de guides est connecté via un connecteur 6 au boîtier 4 de manière à présenter l'extrémité proximale du toron 11 de guides au système de balayage.

Le système de balayage comprend deux miroirs distincts qui dirigent un faisceau lumineux d'excitation tour à tour dans une des fibres des guides depuis l'extrémité proximale du toron 11 de guides, cet éclairement des fibres une par une étant transmis par celles-ci jusqu'à leur extrémité distale où chacune devient à son tour une source d'illumination.

L'extrémité distale de chaque guide d'image 1, 2, ou 3 est connectée à une tête optique 7, 8, ou 9, qui :
- focalise le faisceau lumineux d'excitation, en provenance d'une fibre, en surface ou en profondeur de la zone d'intérêt de l'animal 10 dans laquelle la tête optique est implantée,
- collecte un faisceau lumineux réfléchi ou ré-émis par le tissu biologique illuminé dans cette zone d'intérêt, et
- transmet le faisceau collecté à la même fibre.

Le faisceau lumineux collecté est ensuite transmis depuis l'extrémité distale jusqu'à l'extrémité proximale du guide d'image, et est dirigé à l'intérieur du boîtier 4 jusqu'à un détecteur de faisceaux lumineux.

Le balayage de l'extrémité proximale du toron 11 de guides est périodique, et le boîtier 4 comprend en outre des moyens pour extraire, parmi tous les faisceaux lumineux détectés par le détecteur au cours d'une période, un ensemble de faisceaux lumineux collectés à l'extrémité distale d'un des guides par plusieurs fibres dudit guide. Le boîtier 4 comprend en outre un module de synthèse d'image qui construit une image à partir de l'ensemble de faisceaux. L'écran de visualisation 5 affiche cette image. L'écran de visualisation peut ainsi afficher une image pour chacun des guides d'image 1, 2, ou 3.

Le boîtier 4 comprend en outre un laser émettant le faisceau lumineux d'excitation en mode continu ou pulsé, et des moyens de variation du contenu spectral du faisceau lumineux d'excitation. Les moyens de variation peuvent être programmés pour diriger un faisceau lumineux d'excitation d'une longueur d'onde donnée par guide d'image.

Il existe de nombreuses variantes possibles à partir du premier mode de réalisation de dispositif selon l'invention, notamment en ce qui concerne le nombre de guides, le nombre de fibres par guide, le diamètre des fibres et le diamètre des guides. Par exemple, la variante pour laquelle les extrémités proximales de sept guides sont combinées en un toron de guides s'applique bien au cas de guides de même diamètre.

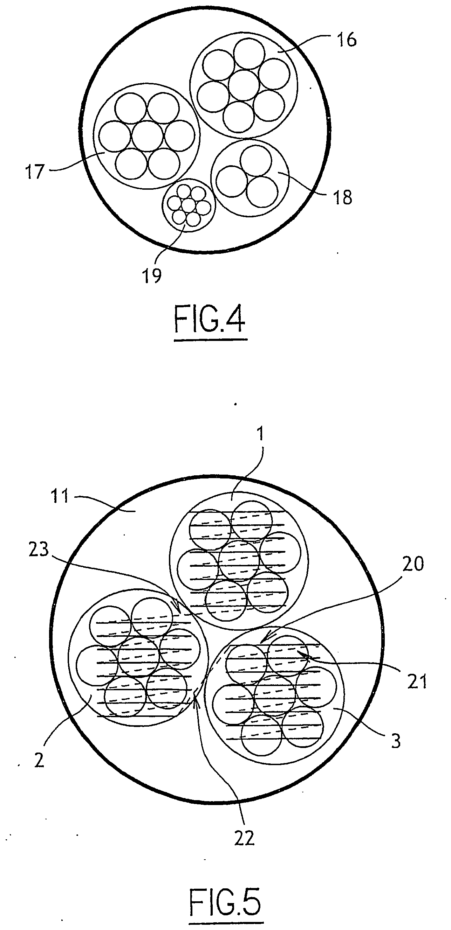

Un second mode de réalisation de dispositif selon l'invention comprend tous les mêmes éléments que le premier mode de réalisation de dispositif selon l'invention, à l'exception du fait qu'il ne comprend pas trois mais quatre guides d'image dont les extrémités proximales sont regroupées en un toron de guides. La figure 4 illustre une vue en coupe du toron de guides du second mode de réalisation de dispositif selon l'invention. Deux premiers guides 16, 17 ont le même nombre de fibres et ont des fibres de même diamètre, mais ne sont pas agencés pour conserver à l'extrémité proximale du toron de guides un ordonnancement des fibres d'un guide à l'autre. Les deux autres guides 18, 19 ont des nombres de fibres ou des diamètres de fibres qui diffèrent des deux premiers guides 16, 17 et possèdent donc chacun un diamètre différent des deux premiers guides.

On va maintenant décrire, en référence aux figures 5, 6, et 7, un premier, un second et un troisième modes de réalisation de procédé mis en oeuvre dans le premier mode de réalisation de dispositif selon l'invention.

Les premier, second et troisième modes de réalisation de procédé comprennent :
- un couplage des trois guides d'image 1, 2, 3 à un système de balayage, et
- un balayage périodique des guides par le système de balayage, le balayage dirigeant un faisceau lumineux d'excitation tour à tour dans une des fibres des guides depuis l'extrémité proximale du guide comprenant ladite une des fibres.

Les premier, second et troisième modes de réalisation de procédé comprennent en outre, pour chaque fibre dans laquelle le faisceau lumineux d'excitation est dirigé tour à tour:
- une transmission du faisceau lumineux d'excitation, le long de la fibre depuis l'extrémité proximale jusqu'à l'extrémité distale du guide comprenant la fibre,
- une focalisation du faisceau d'excitation par la tête optique connectée au guide,
- une collection par la tête optique d'un faisceau lumineux réfléchi ou réémis par diffusion ou par fluorescence,
- une transmission du faisceau lumineux collecté à l'extrémité distale du guide,
- une transmission du faisceau lumineux collecté, le long de la fibre depuis l'extrémité distale jusqu'à l'extrémité proximale du guide, et
- une détection par le détecteur du faisceau lumineux collecté, la fibre étant associée dans une table de correspondance avec les pixels du détecteur qui détectent le faisceau collecté et avec l'instant, au cours de la période de balayage, pendant lequel le faisceau est détecté.

Par conséquence, on dirige un faisceau lumineux d'excitation tour à tour dans une des fibres des guides depuis l'extrémité proximale du guide comprenant ladite une des fibres, et on détecte tour à tour des faisceaux lumineux collectés à l'extrémité distale de cette fibre, cette fibre effectuant donc un filtrage spatial de la lumière collectée à l'extrémité distale du guide. Ainsi, le détecteur du premier mode de réalisation de dispositif selon l'invention est agencé pour détecter un faisceau lumineux collecté à l'extrémité distale d'une fibre d'un des guides en réponse au faisceau d'excitation dirigé dans cette même fibre, cette fibre effectuant donc un filtrage spatial de la lumière collectée à l'extrémité distale du guide, ce qui rend le premier mode de réalisation de dispositif selon l'invention confocal.

La table de correspondance est crée au cours d'une étape de calibration du dispositif selon l'invention, après avoir couplé le toron de guides au système de balayage. Les premier, second et troisième modes de réalisation de procédé comprennent en outre pour chaque guide d'image au cours d'une période:
- une extraction, parmi tous les faisceaux détectés par le détecteur et à l'aide de la table de correspondance, d'un ensemble de faisceaux lumineux collectés par plusieurs fibres à l'extrémité distale du guide et détectés par le détecteur,
- une construction d'une image à partir de l'ensemble de faisceaux lumineux collectés et détectés, l'image représentant un champ d'observation d'une zone d'intérêt dans laquelle la tête optique du guide est implantée, la construction comprenant un traitement adéquat pour éliminer l'empreinte du réseau de milliers de fibres du guide, et
- une visualisation de l'image sur l'écran de visualisation.

Pour les guides d'image 1, 2, 3 comprenant plusieurs milliers de fibres de quelques micromètres de diamètre, la surface typique du champ d'observation est de l'ordre du millimètre carré, cette surface dépendant notamment des caractéristiques de la tête optique disposée sur l'extrémité distale du guide 1, 2 , ou 3. En implantant les têtes optiques dans plusieurs zones d'intérêt d'un animal, le procédé permet donc de visualiser in vivo, in situ et simultanément plusieurs zones d'intérêts de l'animal avec une résolution microscopique.

La figure 5 illustre, sur une vue en coupe de l'extrémité proximale du toron 11 de guides du premier mode de réalisation de dispositif selon l'invention, une période de balayage dans le cas du premier mode de réalisation de procédé. Dans une période de balayage du premier mode de réalisation de procédé, le système de balayage dirige le faisceau lumineux d'excitation tour à tour dans une fibre depuis l'extrémité proximale du toron 11 de guides, le long de lignes horizontales parallèles 20 reliées par des lignes obliques 21. Un ensemble de lignes horizontales 20 reliées par des lignes obliques 21 constituent une trame évoquant la trame d'un écran de télévision. Les lignes horizontales 20 et les lignes obliques 21 sont agencées pour former une trame par guide d'image 1, 2, ou 3. Les lignes horizontales 20 ne dirigent pas le faisceau d'excitation dans des intervalles compris entre les extrémités proximales des guides d'image. La trame du premier guide balayé 1 est reliée à la trame du second guide balayé 2 par une première ligne oblique de relais 23, et la trame du second guide balayé 2 est reliée à la trame du troisième guide balayé 3 par une seconde ligne oblique de relais 22. Au cours d'une période, toutes les fibres sont balayées. Les lignes horizontales 20 sont suffisamment rapprochées pour diriger le faisceau lumineux d'excitation plus d'une fois par période dans une même fibre, ce qui permet une grande résolution d'échantillonnage des fibres. Les moyens techniques actuels de balayage permettent de balayer à cette résolution l'extrémité proximale du toron 11 de guides avec une période d'une centaine de millisecondes. Le temps de balayage d'une trame d'un guide est donc d'une trentaine de millisecondes. Trois images construites au cours d'une période, à partir de trois ensembles de faisceaux collectés provenant chacun d'un guide donné, correspondent donc à une observation quasi-simultanée de trois zones d'intérêt dans lesquelles les trois têtes optiques sont implantées. Le premier mode de réalisation de procédé comprend en outre une variation de longueur d'onde du faisceau lumineux d'excitation, de sorte que le faisceau lumineux d'excitation soit monochromatique et d'une longueur d'onde donnée par guide. Ainsi, chaque guide d'image peut servir à une excitation d'un colorant différent par zone d'intérêt.

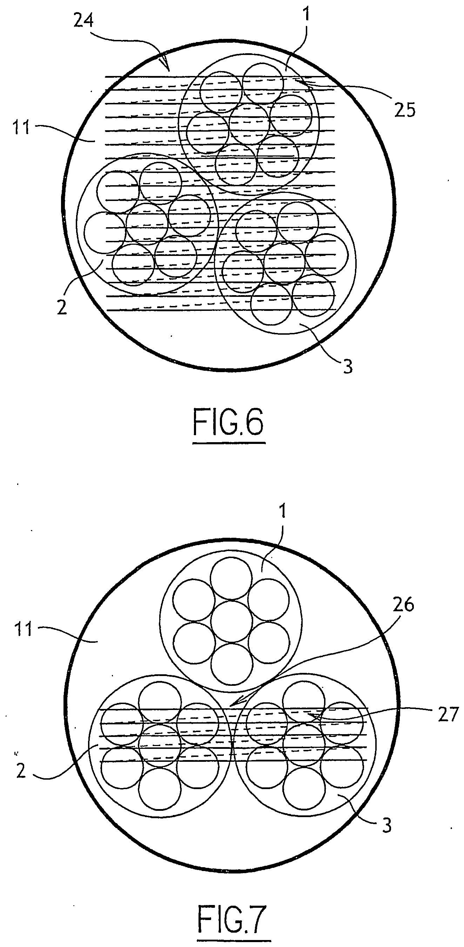

La figure 6 illustre, sur une vue en coupe de l'extrémité proximale du toron 11 de guides du premier mode de réalisation de dispositif selon l'invention, une période de balayage dans le cas du second mode de réalisation de procédé. Dans une période de balayage du second mode de réalisation de procédé, le système de balayage dirige le faisceau lumineux d'excitation tour à tour dans une des fibres depuis l'extrémité proximale du toron 11 de guides, le long de lignes horizontales parallèles 24 reliées par des lignes obliques 25. Les lignes horizontales 24 sont suffisamment rapprochées pour diriger le faisceau lumineux d'excitation plus d'une fois par période dans une même fibre. Au cours d'une période, toutes les fibres sont balayées, et les lignes horizontales dirigent le faisceau lumineux d'excitation en outre dans des intervalles compris entre les extrémités proximales des guides d'image. Les lignes horizontales 24 reliées par des lignes obliques 25 constituent une trame commune aux trois guides 1, 2, 3 qui évoque la trame d'un écran de télévision, de sorte qu'une même ligne horizontale balaye généralement plus d'un guide d'image. Les trois guides d'image sont donc balayés quasi simultanément au cours d'une période, et trois images construites au cours d'une période, à partir de trois ensembles de faisceaux collectés provenant chacun d'un guide donné, correspondent à une observation quasi-simultanée de trois zones d'intérêt dans lesquelles les trois têtes optiques sont implantées.

La figure 7 illustre, sur une vue en coupe de l'extrémité proximale du toron 11 de guides du premier mode de réalisation de dispositif selon l'invention, une période de balayage dans le cas du troisième mode de réalisation de procédé. Dans une période de balayage du troisième mode de réalisation de procédé, le système de balayage dirige le faisceau lumineux d'excitation tour à tour dans une fibre depuis l'extrémité proximale du toron 11 de guides, le long de lignes horizontales parallèles 26 reliées par des lignes obliques 27. Les lignes horizontales 26 sont suffisamment rapprochées pour diriger le faisceau lumineux d'excitation plus d'une fois par période dans une même fibre, mais ne sont pas suffisamment nombreuses pour diriger le faisceau lumineux d'excitation dans les trois guides et dans toutes les fibres des deux guides 2, 3 balayés. Les lignes horizontales 26 reliées par des lignes obliques 27 constituent une trame commune à deux des guides 2 et 3, de sorte qu'une même ligne horizontale 26 balaye généralement les deux guides 2, 3 balayés par la trame. Les deux guides d'image 2, 3 balayés sont donc balayés quasi simultanément durant une période. Balayer au cours d'une période uniquement certaines fibres de certains guides permet de diminuer la période de balayage jusqu'à quelques millisecondes.

Bien sûr, l'invention n'est pas limitée aux exemples qui viennent d'être décrits et de nombreux aménagements peuvent être apportés à ces exemples sans sortir du cadre de l'invention. Dans un dispositif selon l'invention, le nombre de guides d'image utilisés, le diamètre des fibres des guides, et le nombre de fibres par guide peuvent prendre de nombreuses valeurs possibles. Un dispositif selon l'invention peut être appliqué à de nombreux domaines comme la spectroscopie, l'imagerie de fluorescence, de réflectance, ou multiphotonique. Dans le cadre de l'imagerie multiphotonique, un dispositif selon l'invention peut comprendre en outre des moyens de précompensation pour compenser des éventuels effets dispersifs des fibres. Ces moyens de précompensation peuvent être placés aux extrémités proximales des guides d'image, à condition que les guides aient des propriétés de dispersion similaires.

## Revendications

1. Dispositif d'endoscopie comprenant :
- plusieurs guides d'image (1, 2, 3) comprenant chacun deux extrémités respectivement proximale et distale reliées par plusieurs fibres optiques (12), l'extrémité distale de chaque guide étant séparée des extrémités distales des autres guides de sorte que chaque extrémité distale de guide soit mobile indépendamment des autres guides, et
- des moyens de détection d'un faisceau lumineux collecté à l'extrémité distale d'un des guides,
le dispositif comprenant en outre un système de balayage relié optiquement auxdites extrémités proximales et agencé pour diriger un faisceau lumineux d'excitation tour à tour dans une des fibres desdits guides depuis l'extrémité proximale du guide comprenant ladite une des fibres, et en ce que les moyens de détection sont agencés pour détecter un faisceau lumineux collecté par une fibre en réponse au faisceau d'excitation dirigé dans cette même fibre, les extrémités proximales des guides (1, 2, 3) étant collées ensemble et entourées par une gaine de protection (15) de manière à être associées en un toron (11) de guides,
et en ce qu'il comprend en outre un connecteur (6) pour connecter l'extrémité proximale du toron (11) de guides au système de balayage (4)
et en ce qu'il comprend en outre des moyens pour extraire, parmi des faisceaux collectés par un ou plusieurs guides, un ensemble de faisceaux lumineux collectés par plusieurs fibres à l'extrémité distale d'un des guides, les moyens de détection comprenant un détecteur, ces moyens d'extraction comprenant des moyens pour mettre en oeuvre un algorithme d'extraction utilisant une table de correspondance entre chaque fibre et des pixels du détecteur, ou une table de correspondance entre chaque fibre et un instant de la période du balayage si le balayage est périodique, de sorte que les moyens d'extraction associent chaque faisceau détecté par le détecteur avec la fibre d'où provient le faisceau.

2. Dispositif selon la revendication 1, **caractérisé en ce qu'**il comprend en outre une source de lumière pour émettre le faisceau lumineux d'excitation.

3. Dispositif selon l'une des revendications 1 à 2, **caractérisé en ce qu'**il comprend en outre une tête optique (7, 8, 9) disposée sur une extrémité distale d'un des guides (1, 2, 3).

4. Dispositif selon l'une des revendications 1 à 3, **caractérisé en ce qu'**il comprend en outre des moyens pour varier le contenu spectral du faisceau lumineux d'excitation en fonction du guide (1, 2 , 3) dans lequel est dirigé le faisceau lumineux d'excitation.

5. Dispositif selon l'une quelconque des revendications précédentes, **caractérisé en ce qu'**il comprend en outre des moyens pour construire une image à partir de l'ensemble de faisceaux lumineux collectés.

6. Dispositif selon la revendication 5, **caractérisé en ce qu'**il comprend en outre des moyens (5) pour visualiser l'image.

## Patentansprüche

1. Endoskopievorrichtung, enthaltend:
- mehrere Bildleiter (1, 2, 3), die jeweils zwei Enden, ein proximales und ein distales, aufweisen, die über mehrere Lichtleitfasern (12) verbunden sind, wobei das distale Ende eines jeden Leiters von den distalen Enden der weiteren Leiter getrennt ist, so dass jedes distale Leiterende unabhängig von den weiteren Leitern beweglich ist, und
- Detektionseinrichtungen zum Erfassen eines Lichtstrahlenbündels, das am distalen Ende eines der Leiter aufgefangen wird,
wobei die Vorrichtung ferner ein Abtastsystem enthält, das optisch mit den proximalen Enden verbunden und dazu vorgesehen ist, ein Anregungslichtstrahlenbündel nacheinander in eine der Fasern der Leiter von dem die eine Faser enthaltenden proximalen Ende des Leiters einzuleiten,
und wobei die Detektionseinrichtungen dazu vorgesehen sind, ein von einer Faser aufgefangenes Lichtstrahlenbündel in Reaktion auf das in diese gleiche Faser eingeleitete Anregungsstrahlenbündel zu detektieren, wobei die proximalen Enden der Leiter (1, 2, 3) zusammengeklebt sind und von einer Schutzhülle (15) umgeben sind, so dass sie zu einem Strang (11) von Leitern gekoppelt sind,
und wobei sie ferner einen Verbinder (6) aufweist, um das proximale Ende des Strangs (11) der Leiter mit dem Abtastsystem (4) zu verbinden,
und wobei sie ferner Einrichtungen enthält, um aus über einen oder mehrere Leiter aufgefangenen Strahlenbündeln eine Einheit von über mehrere Fasern an einem distalen Ende eines der Leiter aufgefangenen Lichtstrahlenbündeln auszukoppeln, wobei die Detektionseinrichtungen einen Sensor enthalten, wobei diese Auskoppeleinrichtungen Einrichtungen enthalten, um einen Auskoppelalgorithmus durchzuführen, der eine Verknüpfungstabelle zwischen jeder Faser und Pixeln des Sensors oder eine Verknüpfungstabelle zwischen jeder Faser und einem Zeitpunkt der Periode der Abtastung - bei periodisch erfolgender Abtastung - verwendet, so dass die Auskoppeleinrichtungen jedes von dem Sensor erfasste Strahlenbündel mit der Faser koppeln, von welcher das Strahlenbündel stammt.

2. Vorrichtung nach Anspruch 1, **dadurch gekennzeichnet, dass** sie ferner eine Lichtquelle enthält, um das Anregungslichtstrahlenbündel zu emittieren.

3. Vorrichtung nach einem der Ansprüche 1 bis 2, **dadurch gekennzeichnet, dass** sie ferner einen optischen Kopf (7, 8, 9) enthält, der an einem distalen Ende eines der Leiter (1, 2, 3) angeordnet ist.

4. Vorrichtung nach einem der Ansprüche 1 bis 3, **dadurch gekennzeichnet, dass** sie ferner Einrichtungen enthält, um den spektralen Gehalt des Anregungslichtstrahlenbündels in Abhängigkeit von dem Leiter (1, 2, 3) zu variieren, in welchen das Anregungslichtstrahlenbündel eingeleitet wird.

5. Vorrichtung nach einem der vorangehenden Ansprüche, **dadurch gekennzeichnet, dass** sie ferner Einrichtungen enthält, um ein Bild ausgehend von der Einheit von aufgefangenen Lichtstrahlenbündeln zu konstruieren.

6. Vorrichtung nach Anspruch 5, **dadurch gekennzeichnet, dass** sie ferner Einrichtungen (5) enthält, um das Bild zu visualisieren.

## Claims

1. Endoscopy device comprising:
- several image guides (1, 2, 3) each comprising two ends, respectively proximal and distal, linked a plurality of optical fibres (12), the distal end of each guide being separated from the distal ends of the other guides so that each distal end of a guide can move independently of the other guides,
- detection means arranged for detecting a light beam collected at the distal end of one of the guides
the device further comprising a scanning system optically linked to said proximal ends and arranged to direct an excitation light beam in turn into one of the fibres of said guides from the proximal end of the guide comprising said one of the fibres, the detection means being arranged for detecting a light beam collected by a fibre in response to the excitation beam directed into this same fibre, the proximal ends of the guides (1, 2, 3) being sticked together and surrounded by a protective sheath (15) in order to be combined to form a bundle (11) of guides,
the device also comprising a connector (6) for connecting the proximal end of the bundle (11) of guides to the scanning system (4)
the device also comprising means for extracting, from beams collected by one or more guides, a set of light beams collected by several fibres at the distal end of one of the guides, the detection means comprising a detector, these extraction means comprising means for implementing an extraction algorithm using a correspondence table between each fibre and pixels of the detector, or a correspondence table between each fibre and a moment of the scanning period if the scanning is periodic, such that the extraction means associate each beam detected by the detector with the fibre from which the beam originates.

2. Device according to claim 1, **characterized in that** it also comprises a light source for emitting the excitation light beam.

3. Device according to one of claims 1 to 2, **characterized in that** it also comprises an optical head (7, 8, 9) arranged on a distal end of one of the guides (1, 2, 3).

4. Device according to one of claims 1 to 3, **characterized in that** it also comprises means for varying the spectral content of the excitation light beam depending on the guide (1, 2 , 3) into which the excitation light beam is directed.

5. Device according to any of the previous claims, **characterized in that** it also comprises means for constructing an image from the set of collected light beams.

6. Device according to claim 5, **characterized in that** it also comprises means (5) for displaying the image.
